# EUROPÄISCHE PATENTSCHRIFT

(11) **EP 2 044 884 B1**
(45) Veröffentlichungstag und Bekanntmachung des Hinweises auf die Patenterteilung: **09.12.2015**
(21) Anmeldenummer: 07117756.2
(22) Anmeldetag: 02.10.2007
(51) Int. Cl.: A61B 5/103, A61B 8/08, A61B 19/00, G01R 33/565, A61B 5/11, A61B 5/107, G06F 19/00

(54) **Bestimmung und Erkennung von Lageänderungen von Körperstrukturteilen**
Detection and determination of changes in position of structural parts of a body
Détermination et détection de modifications de position de parties de structure corporelle

(43) Veröffentlichungstag der Anmeldung: 08.04.2009
(73) Patentinhaber: Brainlab AG, 85622 Feldkirchen (DE)
(72) Erfinder: Vollmer, Fritz, 80469 München (DE); Leidel, Martin, 83714 Miesbach (DE); Thiemann, Ingmar, 80686 München (DE)
(74) Vertreter: Schwabe - Sandmair - Marx

(56) Entgegenhaltungen:
- WO-A-2005/025404
- DE-A1- 10 333 543
- DE-A1- 19 751 761
- LETTEBOER M M J ET AL: "Brain Shift Estimation in Image-Guided Neurosurgery Using 3-D Ultrasound" IEEE TRANSACTIONS ON BIOMEDICAL ENGINEERING, IEEE SERVICE CENTER, PISCATAWAY, NJ, US, Bd. 52, Nr. 2, Februar 2005 (2005-02), Seiten 268-276, XP011125712 ISSN: 0018-9294
- MAINTZ J B A ET AL: "A SURVEY OF MEDICAL IMAGE REGISTRATION" MEDICAL IMAGE ANALYSIS, OXFORDUNIVERSITY PRESS, OXFORD, GB, Bd. 2, Nr. 1, 1998, Seiten 1-37, XP001032679 ISSN: 1361-8423
- TORIL A NAGELHUS HERNES ET AL: "Computer-assisted 3D ultrasound-guided neurosurgery: technological contributions, including multimodal registration and advanced display, demonstrating future perspectives" INTERNATIONAL JOURNAL OF MEDICAL ROBOTICS AND COMPUTER ASSISTED SURGERY, JOHN WILEY, CHICHESTER, GB, Bd. 2, 2006, Seiten 45-49, XP007904393 ISSN: 1478-5951
- I-A RASMUSSEN ET AL: "Functional neuronavigation combined with intra-operative 3D ultrasound: Initial experiences during surgical resections close to eloquent brain areas and future directions in automatic brain shift compensation of preoperative data" ACTA NEUROCHIRURGICA ; THE EUROPEAN JOURNAL OF NEUROSURGERY, SPRINGER-VERLAG, VI, Bd. 149, Nr. 4, 19. Februar 2007 (2007-02-19), Seiten 365-378, XP019519267 ISSN: 0942-0940

## Beschreibung

Operationen werden vom Arzt vorzugsweise mittels so genannter präoperativer Daten (beispielsweise Kernspin oder CT-Daten) geplant. Gerade bei der Operation von Weichteilen (z.B. Gehirn oder Leber) kann es während der Operation zu einer Verschiebung der Weichteile (die ein Beispiel für eine Körperstruktur darstellen) relativ zu anderen Körperteilen (z.B. Knochen) kommen. Die vorliegende Erfindung betrifft insbesondere die navigationsunterstützte Chirurgie, bei der präoperative Daten über die Körperstruktur vorliegen, die die relative Lage der Körperstruktur relativ zu Markern beschreiben. Die Lage zumindest eines Teils der Körperstruktur kann sich aber, wie erwähnt, während der Operation relativ zu den Markern verschieben. Diese Verschiebung oder Verlagerung kann durch intraoperative Analyseverfahren (z.B. Ultraschall oder Röntgenaufnahmen) erkannt werden, die sowohl zumindest einen Teil der Körperstruktur als auch die Marker erfassen.

Aufgabe der Erfindung ist es, Lageänderungen automatisch zu bestimmen oder das Erkennen einer Lageänderung dem Bediener zu erleichtern.

Vorstehende Aufgabe wird durch die Gegenstände der unabhängigen Ansprüche gelöst. Vorteilhafte Weiterbildungen gehen aus den abhängigen Ansprüchen hervor.

Das erfindungsgemäße Verfahren dient vorzugsweise der Bestimmung und/oder dem Erkennen einer Lageänderung eines Teils einer Körperstruktur relativ zu einer Markereinrichtung. Eine Körperstruktur kann insbesondere Weichteile und Knochen umfassen. Ein Beispiel für eine Körperstruktur stellt der Kopf dar. Eine Markereinrichtung ist vorzugsweise ortsfest mit der Körperstruktur (z.B. starren Körperstrukturteilen, wie Knochen) verbunden. Beispielsweise umfasst die Markereinrichtung einen Referenzstern und/oder mehrer Markerelemente (Markerkugeln). Die Markerelemente können beispielsweise aktiv ausgebildet sein, so dass sie Strahlen und/oder Wellen aussenden (beispielsweise Licht, insbesondere Infrarotlicht). Sie können auch passiv ausgebildet sein, so dass sie Wellen DE 103 33 543 A2 offenbart ein Verfahren zur gekoppelten Darstellung präoperativ und intraoperativ aufgenommener Bilder insbesondere zur Bestimmung des brain-shifts. Eine visuelle Überprüfung durch den Anwender, ob und inwieweit eine Übereinstimmung eines präoperativen Bilds mit einem intraoperativen Bild vorliegt, ist vorgesehen.

Letteboer et al. "Brain Shift Estimation in Image-Guided Neurosurgery Using 3-D Ulttrasound", IEEE Transactions on Biomedical Engineering, Vol. 52, No. 2, Februar 2005, S. 268-276 offenbart ein Verfahren zur Bestimmung der Verschiebung des Gehirns durch Vergleich der Grenzen des Gehirns in einem präoperativen MR-Bild und einem intraoperativen Ultraschallbild.

Maintz und Viergever, A Survey of Medical Image Registration, Medical Image Analysis, Oxford University Press, Oxford, Bd. 2, Nr. 1, 1998, S. 1-37 offenbart Verfahren zur Identifizierung einander entsprechender anatomischer Strukturen.

DE 197 517 61 A1 offenbart ein System zur Erfassung von Behandlungszielpunkten unter Verwendung von Reflektoren, Markern und Positionsdetektionseinrichtungen.

WO 2005/025404 A2 offenbart eine Vorrichtung und ein Verfahren zur Registrierung der Kortexoberfläche und Bestimmung einer Deformation der Gehimoberfläche, wobei eine farbliche Hervorhebung bestimmter Punktwolken in einer bildlichen Darstellung vorgeschlagen wird.

Nagelhus Hernes et al., Computer-assisted 3D Ultrasound-Guided Neurosurgery: Technological Contributions, including Multimodal Registration and Advance Display, Demonstrating Future Perspectives, Int J Med Robotics Comput Assist Surg 2006; 2: 45-59 offenbart ein Verfahren zur Bestimmung einer Verschiebung des Gehirns mittels Vergleichs von präoperativen MRI-Daten und intraoperativen Ultraschalldaten, wobei ein Registrieralgorithmus ohne Verwendung von Markem vorgeschlagen wird.

Rasmussen Jr. et al., Functional Neuronavigation Compliant with Intra-Operative 3D Ultrasound: Initial Experiences During Surgical Resections Close to Eloquent Brain Areas and Future Directions in Automatic Brain Shift Compensation of Preoperative Data, Acta Neurochir (Wien), 2007, 149: 365-378 offenbart ein Registrierverfahren zur automatischen Bestimmung der Gehirnverschiebung aus präoperative MR-Daten und intraoperativen 3D-Ultraschalldaten unter Verwendung automatischer voxelbasierter Registrierung. und/oder Strahlen reflektieren, also insbesondere Licht (z.B. Infrarotlicht) reflektieren, das beispielsweise von einer Strahlen- und/oder Wellenquelle (z.B. Lichtquelle) ausgesendet wird. Die passiv reflektierten Wellen und/oder Strahlen oder die aktiv emittierten Wellen und/oder Strahlen werden vorzugsweise von einer Detektionseinrichtung detektiert, die die Lage der Markereinrichtung somit erfassen kann. Insbesondere nehmen die Markerelemente eine bestimmte relative Lage zueinander ein.

Erfindungsgemäß werden präoperative Daten und intraoperative Daten der Körperstruktur bereitgestellt. Die präoperativen Daten unterscheiden sich von den intraoperativen Daten insbesondere darin, dass sie früher gewonnnen wurde. Insbesondere sind die präoperativen Daten in ihrem Informationsgehalt umfangreicher als die intraoperativen Daten. Üblicherweise steht für ein präoperativ angewendetes medizinisches Analyseverfahren, wie beispielsweise ein Kernspin- oder dreidimensionales Röntgen-CT-Verfahren, ein längerer Zeitraum zur Verfügung, um Informationen zu sammeln. Insbesondere ist die räumliche Auflösung der Daten, die die räumliche Struktur der Körperstruktur beschreiben, im Falle der präoperativen Daten feiner als im Falle der intraoperativen Daten. Auch können sich die präoperativen Daten von den intraoperativen Daten darin unterscheiden, dass sie -von verschiedenen medizinischen Analyseeinrichtungen gewonnen wurden. Intraoperativ können z.B. Ultraschalldiagnosegeräte eingesetzt werden, und die so gewonnenen Daten können mit präoperativ gewonnnen Kernspindaten durch das erfindungsgemäße Verfahren verarbeitet werden.

Vorzugsweise beschreiben sowohl die präoperativen Daten als auch die intraoperativen Daten die Lage zumindest eines bestimmten Teils der Körperstruktur (oder auch der gesamten Körperstruktur) relativ zur Markereinrichtung. Vorzugsweise wird basierend auf den präoperativen Daten und den intraoperativen Daten bestimmt, ob die Lage des durch die präoperativen Daten beschriebenen zumindest einen bestimmten Teils der Körperstruktur von der Lage des durch die intraoperativen Daten beschriebenen (selben) Teils der Körperstruktur verschieden ist. Diese Bestimmung erfolgt insbesondere automatisch und ist vorzugsweise insbesondere dann mit der automatischen Ausgabe einer Hinweisinformation verknüpft, wenn eine Lageänderung des Teils von der präoperativen zur intraoperativen Situation, also eine unterschiedliche Lage festgestellt wird. Eine entsprechende Feststellung kann natürlich auch für mehrere, verschiedene Teile der Körperstruktur vorgenommen werden. Im Folgenden wird die Erfindung rein beispielhaft anhand eines Teiles (oder einer Substruktur) beschrieben, das seine Lage in der Zeit zwischen der Erfassung der präoperativen und intraoperativen Daten ändert.

Alternativ oder zusätzlich erfolgt erfindungsgemäß vorzugsweise die Anzeige, insbesondere automatische Anzeige zumindest des Teils der Körperstruktur, wie sie sich aus den intraoperativen Daten und aus den präoperativen Daten ergibt. Die Anzeige erfolgt vorzugsweise so, dass ein Bediener leicht erkennen kann, ob die Lage des präoperativen Teils sich von der Lage des (entsprechenden) intraoperativen Teils unterscheidet. Insbesondere wird die Lage des präoperativen Teils und des (entsprechenden) intraoperativen Teils beispielsweise überlagert gezeigt oder nebeneinander oder untereinander, wobei in den letzteren Fällen vorzugsweise die Anzeige mit einer Skalierung unterlegt ist, um so leichter eine Lageverschiebung mit dem Auge feststellen zu können. Insbesondere bei einer überlagerten Anzeige beider Teile können beide Teile verschiedenfarbig angezeigt werden. Die automatische Bestimmung, ob eine Lageänderung stattgefunden hat, also ob sich die Lagen unterscheiden, kann insbesondere mit der automatischen Anzeige verbunden werden. Die Verbindung kann insbesondere so erfolgen, dass diejenigen Teile bildlich (z.B. durch Farbe oder Blinken) hervorgehoben werden, die eine Lageänderung erfahren haben.

Vorzugsweise werden das präoperative Teil und/oder das intraoperative Teil durch so genannte (anatomische) Objekte dargestellt, die sich durch Anwendung von Bildverarbeitungsalgorithmen (z.B. zur Objektextraktion, zur Glättung oder zur Kantendetektion) aus den präoperativen bzw. intraoperativen Daten ergeben.

Vorzugsweise werden die den präoperativen und intraoperativen Daten entsprechenden Bilddaten skaliert und vorzugsweise in demselben Koordinatensystem ausgegeben, wobei insbesondere die Marker, die nicht notwendigerweise gezeigt werden, in beiden Koordinatensystemen dieselbe Lage einnehmen, so dass der Bediener bei der Anzeige der Bilddaten einen Vergleich der Lage vornehmen kann. Dieser Vergleich wird, wie bereits oben ausgeführt, vorzugsweise durch die Anzeige einer Skala erleichtert.

Erfolgt eine automatische Bestimmung, ob die Lage des präoperativen Teils von der Lage des intraoperativen Teils verschieden ist, so kann dies basierend darauf erfolgen, dass ein Bediener z.B. interaktiv in den Bilddaten sowohl das präoperative Teil als auch das intraoperative Teil bezeichnet, indem er es beispielsweise mit einer Maus umfährt. Vorzugsweise erfolgt eine Bestimmung des präoperativen Teils und/oder des intraoperativen Teils automatisch.

Man nehme an, der Bediener hat in den präoperativen Daten einen bestimmten Bereich als für die Operation relevant geplant. Dieser bestimmte Bereich enthält beispielsweise ein präoperatives Teil, also beispielsweise einen Tumor im Gehirn. Der Bediener kann dann das präoperative Teil so bestimmen, indem er dessen Grenzen z.B. mit einer Maus festlegt. Alternativ können z.B. Bildverarbeitungsalgorithmen z.B. aus einem grob bezeichneten Bereich eine zusammenhängende Struktur extrahieren, die dann das präoperative Teil der Körperstruktur (oder eine präoperative Substruktur) darstellt, deren Form und/oder Lageänderung erfindungsgemäß bestimmt oder erkannt werden soll. Vorzugsweise wird basierend auf den intraoperativen Daten und basierend auf dem bestimmten präoperativen Teil automatisch das entsprechende intraoperative Teil bestimmt.

Die Entsprechung des präoperativen Teils mit dem intraoperativen Teil (oder umgekehrt) kann beispielsweise basierend auf der Form des präoperativen Teils und/oder intraoperativen Teils erfolgen. Vorzugsweise umfassen deshalb die präoperativen Daten und die intraoperativen Daten Informationen über die Form des Teils der Körperstruktur. Beispielsweise kann aus den präoperativen Daten eine gekrümmte oder runde Form des präoperativen Teils ableitbar sein und eine entsprechende gekrümmte oder runde Form kann dann in den intraoperativen Daten gesucht werden, um somit automatisch das dem präoperativen Teil entsprechende intraoperative Teil zu bestimmen. Dies kann natürlich auch für mehrere Teile durchgeführt werden. Insbesondere können hierzu Matching- bzw. Mustererkennungsverfahren eingesetzt werden, um sich entsprechende Teile basierend auf den präoperativen Daten und intraoperativen Daten zu bestimmen. Zusätzlich oder alternativ können beispielsweise basierend auf den präoperativen Daten die Ergebnisse einer medizinischen Analyse simuliert werden, die für die Gewinnung der intraoperativen Daten eingesetzt wird. Diese simulierten Ergebnisse können dann mit den intraoperativen Daten beispielsweise wieder unter Einsatz von Matching- oder Mustererkennungsverfahren verglichen werden, um so sich entsprechende Teile der Körperstruktur zu identifizieren.

Alternativ und insbesondere ergänzend kann bei der automatischen Bestimmung sich entsprechender Teile auf die Lageinformation zurückgegriffen werden. Insbesondere kann eine Wahrscheinlichkeitsrechnung durchgeführt werden, wonach die Entsprechung um so wahrscheinlicher ist, je mehr sich die Lage der potentiell sich entsprechenden Teile relativ zur Lage der Markereinrichtung ähnelt. Insbesondere können bei der Bestimmung einer Entsprechung basierend auf Form und/oder Lage diese Wahrscheinlichkeitsprinzipien, beispielsweise die Prinzipien der Maximum-Entropie-Methode eingesetzt werden. Dabei kann nicht nur auf Information basierend auf einer relativen Lage zur Markereinrichtung zurückgegriffen werden, sondern auch basierend auf einer relativen Lage zu anderen Teilen (z.B. Knochenabschnitten oder Landmarken).

Insbesondere können Verfahren zur elastischen Deformation basierend auf der Maximierung gegenseitiger Information eingesetzt werden. Hierbei ist die Entropie eine der Möglichkeiten für die Informationsüberbestimmung, die bei elastischen Registrierungen maximiert wird.

Vorzugsweise wird nicht nur über die Lageänderung, insbesondere über den Umfang einer Lageänderung mittels einer Hinweisinformation informiert, sondern alternativ und insbesondere zusätzlich auch über eine Formänderung, die durch einen Vergleich der präoperativen Form mit der intraoperativen Form bestimmt wird. Insbesondere kann die Hinweisinformation so gestaltet werden, dass auch über den Umfang der Formänderung informiert wird, indem beispielsweise Abschnitte des Teils, die einer starken Formänderung unterzogen wurden, bildlich (z.B. durch Farbe oder Blinken) hervorgehoben werden.

Vorzugsweise erfolgt eine Anzeige von Bilddaten basierend auf den präoperativen Daten. Gemäß einer Ausführungsform der Erfindung kann diese Anzeige so gestaltet werden oder geändert werden, dass die Lage und/oder Form des präoperativen Teils entsprechend der Lage und/oder Form des intraoperativen Teils geändert wird. Dabei bleiben insbesondere die übrigen Bilddaten unverändert, so dass sich beispielsweise eine Lageverschiebung oder Formänderung des präoperativen Teils relativ zu der übrigen (präoperativ bestimmten) Körperstruktur ergibt und auf einer Anzeige erkennbar wird. Vorteilhaft ist diese Vorgehensweise insbesondere dann, wenn die präoperativen Daten detailgetreuer sind und insbesondere leicht erkennbar ist, ob sich das präoperative Teil nun näher an für die Operation kritischen Stellen befindet oder nicht. Beispielsweise kann so erkannt werden, ob ein Tumor seine Lage so geändert hat, dass er nun in der Nähe eines empfindlichen Teils des Gehirns (beispielsweise Sprachzentrum) gelangt ist oder nicht. Vorteilhaft wird die präoperativ gewonnene Information durch die intraoperativen Daten ergänzt. Dies insbesondere dann, wenn sich das intraoperative Analyseverfahren von präoperativen unterscheidet und somit andere Aspekte und (physikalische/chemische) Parameter der Körperstruktur erfasst.

Natürlich kann eine Lageverschiebung erfindungsgemäß auch für mehrere Teile angezeigt und beobachtet werden.

Insbesondere bei den intraoperativen Analyseverfahren kann es sein, dass diese nur einen Unterteil einer Substruktur der Körperstruktur, z.B. nur den Rand einer Substruktur (z.B. Tumor) erfassen. Beispielsweise kann es bei der Ultraschalluntersuchung dazu kommen, dass nur ein Teil des Randes eines Tumors erfasst wird und somit basierend auf den intraoperativen Daten als intraoperativer Teil erkannt werden kann. Der intraoperative Teil (z.B. Randabschnitt des Tumors) ist somit nur ein Unterteil einer Substruktur (z.B. Tumor). Erfindungsgemäß wird vorzugsweise nicht nur über eine Lageänderung des Unterteils einer Substruktur informiert, sondern basierend auf der Annahme, dass mit der Lageänderung des Unterteils auch die gesamte Substruktur ihre Lage ändert, über die Lageänderung der Substruktur informiert. Beispielsweise wird hierüber so informiert, dass bei einer Bilddarstellung basierend auf den präoperativen Daten nicht nur die Lageänderung des Unterteils einer Substruktur angezeigt wird, sondern die Lageänderung der gesamten Substruktur. Alternativ oder zusätzlich kann diejenige Substruktur, die unter der vorgenannten Annahme die Lage verändert hat, bildlich hervorgehoben werden (z.B. durch besondere Farbgestaltung oder durch Blinken). Insbesondere wird erfindungsgemäß basierend auf der Lageänderung des intraoperativen Teils relativ zum präoperativen Teil eine entsprechende Lageänderung der Substruktur berechnet und diese Lageänderung durch eine Hinweisinformation angezeigt. Die Hinweisinformation kann beispielsweise eine Anzeige der präoperativen Substruktur bei ihrer präoperativen Lage und eine weitere Anzeige der intraoperativen Substruktur bei ihrer intraoperativen Lage sein, wobei jeweils zusätzlich eine Skala angezeigt wird, damit der Bediener den Umfang der Lägeänderung erkennen kann. Alternativ kann eine überlagerte bildliche Darstellung der präoperativen Lage der Substruktur und der intraoperativen Lage der Substruktur gewählt werden. Alternativ kann beispielsweise auch nur die präoperative Lage oder nur die intraoperative Lage der Substruktur angezeigt werden und über einen Farbcode kenntlich gemacht werden, wie stark die berechnete Lageänderung ausfällt.

Durch die vorgenannte Anzeige des präoperativen Teils und des intraoperativen Teils und/oder der präoperativen Substruktur und der intraoperativen Substruktur lassen sich insbesondere auch Formänderungen erkennen, die sich zwischen der präoperativen und der intraoperativen Situation (Datengewinnung) ergeben haben.

Neben der Anzeige des intraoperativen Teils und des präoperativen Teils oder der intraoperativen Substruktur und der präoperativen Substruktur können auch durch Anwendung von Bildverarbeitungsalgorithmen davon abgeleitete (anatomische) Objekte dargestellt werden.

Vorzugsweise wird die Formänderung der Substruktur basierend auf bestimmten Eigenschaften der Substruktur zumindest für diejenigen Abschnitte der Substruktur berechnet, für die keine intraoperativen Daten vorliegen. Insbesondere kann man bei der Berechnung der Formänderung davon ausgehen, dass die Substruktur bestimmte physikalische Eigenschaften hat, die sich beispielsweise in einer gewissen Plastizität oder Elastizität der Substruktur ausdrücken. Alternativ oder zusätzlich kann davon ausgegangen werden, dass sich bestimmte geometrische Eigenschaften der Substruktur von der Zeit der Erfassung der präoperativen Daten zu der Zeit der Erfassung der intraoperativen Daten nicht oder nur in einem gewissen Umfang geändert haben. Beispielsweise kann man davon ausgehen, dass das Volumen oder eine Fläche, die die Substruktur einnimmt oder umgibt, konstant geblieben ist oder sich nur in einem bestimmten Umfang beispielsweise kleiner als 20 % oder kleiner als 10 % geändert hat. Basierend auf dieser Annahme kann man dann die vermutliche intraoperative Form der Substruktur auch für diejenigen Abschnitte berechnen, für die basierend auf den intraoperativen Daten keine Informationen vorliegen. Beispielsweise kann man eine bestimmte Elastizitätskonstante oder einen bestimmten Bereich für eine Substruktur (z.B. Tumor) annehmen und weiter kann man annehmen, dass sich Kräfte oder Druckeinwirkungen auf den Tumor intraoperativ in einem bestimmten Umfang ändern können, und somit beispielsweise eine Obergrenze für die Verformung annehmen. Als weitere Randbedingung wirkt natürlich derjenige Abschnitt der Substruktur, für den intraoperativ Daten gewonnen wurden und für den somit Information über die Form dieses Abschnitts vorliegt. Falls man beispielsweise basierend auf diesen Informationen und unter der Annahme eines konstanten Volumens zu einer Verformung der Substruktur kommt, die mit den angenommenen Bereichen für Elastizitätskonstante und Kraft- und/oder Druckeinwirkung nicht in Einklang zu bringen sind, so kann ein Warnhinweis ausgegeben werden. Dieser kann dann darauf hinweisen, dass die berechnete Form der Substruktur außerhalb des durch die intraoperativen Daten abgesicherten Abschnitts möglicherweise nicht korrekt ist.

Die Erfindung betrifft weiter ein Programm, das das vorgenannte Verfahren auf einem Computer ausführt. Das Programm umfasst insbesondere Softwareschnittstellen zur Aufnahme der präoperativen oder intraoperativen Daten, um diese dann entsprechend dem Verfahren verarbeiten zu können.

Weiter ist die vorliegende Erfindung auf eine Vorrichtung gerichtet, die zum Bestimmen und/oder zum Erkennen einer Lageänderung des Teils der Körperstruktur relativ zur Markereinrichtung ausgebildet ist. Die Vorrichtung kann Bestandteil eines medizinischen Navigationssystems sein, wie es insbesondere bei der bildgeführten Operation (image guided surgery) angewendet wird. Die Vorrichtung umfasst vorzugsweise eine Markerdetektionseinrichtung zum Detektieren der Markereinrichtung, insbesondere zum intraoperativen Detektieren der Markereinrichtung. Weiter kann die Vorrichtung auch die medizinische Analyseeinrichtung umfassen (oder beispielsweise Daten von dieser erhalten), die intraoperativ zum Gewinnen der intraoperativen Daten eingesetzt wird. Diese medizinische Analyseeinrichtung ist vorzugsweise so ausgebildet, dass sie sowohl die Lage des Teils der Körperstruktur erfassen kann als auch die Lage der Markereinrichtung. Weiter ist vorzugsweise eine Datenspeichereinrichtung, wie beispielsweise Festplatte oder ROM vorgesehen, um die präoperativen Daten der Körperstruktur zu speichern, die beispielsweise über Datenschnittstellen die präoperativen Daten erhält. Datenschnittstellen sind beispielsweise Schnittstellen zum Internet oder Wechselspeicherlaufwerke. Bei der Datenverarbeitungseinrichtung kann es sich beispielsweise um einen herkömmlichen Computer handeln.

Ein Vorteil der Erfindung liegt darin, dass ein Bediener (beispielsweise ein Chirurg) mögliche Verschiebungen von Teilen einer Körperstruktur im Vergleich zur vorher durchgeführten Planung, die auf den präoperativen Daten basierte, erkennen kann, indem er sich das angezeigte Bild ansieht oder angezeigte Bilder vergleicht, wobei eines beispielsweise auf den präoperativen Daten beruht und das andere auf den intraoperativen Daten. Bevorzugt ist eine überlappende Darstellung der präoperativen und intraoperativen Daten. Bei Ultraschallbildern werden vorteilhaft die vorab geplanten Grenzen der Teile der Körperstruktur (Objektgrenzen) in dem intraoperativen Ultraschallbild angezeigt, um so Verschiebungen darzustellen.

Neben der Ultraschalldiagnostik kann beispielsweise auch das so genannte iMRI (diffusion weighted MRI images) eingesetzt werden, um beispielsweise Nervenbahnen zu visualisieren.

Ein iMRI ist ein integriertes MR-Gerät, das während einer OP verwendet werden kann. Es kann für normale MR-Aufnahmen verwendet werden oder auch für DiffusionWeightedImaging oder Diffusion-TensorImaging (DTI). Aus einer diffusionsgewichteten MR-Aufnahmeserie lassen sich Informationen gewinnen, die es ermöglichen, die Diffusionseigenschaften des Gewebes und dadurch insbesondere den Verlauf von Nervenfasern im Gehirn darzustellen.

Die intraoperativ erfassten Nervenbahnen stellen Beispiele für einen Teil einer Körperstruktur dar. Sie können mit präoperativen (üblicherweise höher auflösenden) Daten über den Verlauf von Nervenbahnen erfindungsgemäß insbesondere automatisch verglichen werden. Insbesondere wird erfindungsgemäß die quantitative Beurteilung der Verschiebung oder Verformung erleichtert.

Erfindungsgemäß werden also intraoperativ Daten gewonnen, z.B. mittels navigierten dreidimensionalen Ultraschallmessgeräten oder intraoperativ gewonnenen CT/MRI-Daten, um insbesondere die Lage und/oder Form eines Teils der Körperstruktur (beispielsweise Tumor, Läsion, Nervenfasern...) zu detektieren. Insbesondere kann präoperativ das Teil der Körperstruktur im Rahmen einer Planung bestimmt werden.

Vorteilhaft werden die Grenzen des Teils der Körperstruktur bestimmt, indem insbesondere modalitätsinhärente Merkmale verwendet werden. So stellt sich (Tumor-)Gewebe in verschiedenen Modalitäten (bei verschiedenen Messtechniken) unterschiedlich dar. Manche Tumore sind in Ultraschall gut, manche gar nicht sichtbar. Tumorgrenzen bilden sich in Ultraschall eventuell besser ab als der Tumor selbst, usw. Gehirngewebe ist je nach Aufnahmemodalität im MR unterschiedlich sichtbar (T1/T2). Beispielsweise können auch Nervenbahnen für DTI (Diffusions-Tensor-Bildgebung bzw. diffusion tensor imaging) aus präoperativen und intraoperativen MRI-Bilddaten berechnet werden. Der Vorteil ist, die Deformation während der OP anhand der Nervenbahnen zu bestimmen. Man kann vorteilhaft die Nervenfasern vor und während der OP vergleichen und dadurch eventuell auf Gewebeverschiebungen rückschließen. DTI kann mit einem intraoperativen MR ausreichender Ausstattung genauso erstellt werden wie präoperativ. Da die Faserverbindungen zu bzw. von funktionalen Arealen wichtig sind, ist eine Information über deren Verlauf während der OP vorteilhaft. Ein Vorteil der Erfindung ist, aus der Visualisierung der Veränderung der Nervenfasern Rückschlüsse auf generelle Gewebeverschiebung zu bekommen.

Eine Erkennung von Teilen der Körperstruktur, genauer eine Erkennung, welche Teile der Körperstruktur, die durch die intraoperativen Daten dargestellt werden, Teilen entsprechen, die durch präoperative Daten dargestellt werden, kann man insbesondere wie folgt durchführen:
1. Die Form des präoperativen Teils (also des sich aus den präoperativen Daten ergebenden Teils) kann als Muster oder Schablone verwendet werden, um ein entsprechendes Teil in den intraoperativen Daten zu identifizieren und zu finden.
2. Kennt man die intraoperativen Abbildungsbedingungen, insbesondere die intraoperativ verwendeten medizinischen Analyseverfahren, so kann man basierend auf deren spezifischen Eigenschaften das Muster oder die Schablone vorab entsprechend gestalten. Beispielsweise findet bei Ultraschallaufnahmen unter Umständen eine Abschattung der Seite eines Teils statt, die dem Ultraschallempfänger abgewandt ist. Die Abschattung kann, muss aber nicht stattfinden. Diese Abschattung kann simuliert werden, um so ein optimiertes Muster (oder Schablone) zur Identifizierung und zum Auffinden eines entsprechenden Teils in den intraoperativen Daten zu erleichtern. Anders ausgedrückt kann basierend auf den Eigenschaften der intraoperativ eingesetzten medizinischen Abbildungsverfahren (Analyseverfahren) ein Muster erzeugt werden, für das sich eine bessere Übereinstimmung mit dem aus den intraoperativen Daten abgeleiteten Teil ergibt, als für das präoperative Teil. So kann man die Wahrscheinlichkeit der Erkennung einer Entsprechung zwischen dem intraoperativen Teil und dem präoperativen Teil erhöhen.
3. Beispielsweise können Kantendetektionsfilter auf die präoperativ und/oder intraoperativ gewonnenen Daten angewendet werden, um so die Grenzen des Teils der Körperstruktur (Objektgrenzen) zu identifizieren. Unter Annahme elastischer Objekteigenschaften, kann von (Teilen des) Randes auf das ganze Objekt geschlossen werden.
4. Insbesondere zur automatischen Bestimmung der Lageänderung kann das Muster (Teil-Muster oder Objekt-Muster) insbesondere dreidimensional insbesondere in dem durch die intraoperativen Daten definierten Raum bewegt werden, bis eine Übereinstimmung zwischen dem Teil-Muster und dem intraoperativen Teil optimiert ist, insbesondere bis eine Übereinstimmung der Kanten oder Grenzen des Teil-Musters und der intraoperativ detektierten Kanten bzw. Grenzen maximiert ist. Ist dies der Fall, so wird basierend darauf die Lageverschiebung des Teils der Körperstruktur bestimmt. In einem zweiten Schritt kann dann eine eventuelle Formänderung bestimmt werden, indem beispielsweise stetig die Form des Teil-Musters geändert wird, bis sich eine optimale Übereinstimmung der Formen ergibt. Insbesondere kann mittels eines parametrischen, elastischen Modells die Form iterativ geändert wird, bis eine maximale Überdeckung der Formen erzielt wird.

Das erfindungsgemäße Verfahren, insbesondere das erfindungsgemäße Programm oder die erfindungsgemäße Vorrichtung kann (im Anschluss) die Lageänderung des Teils der Körperstruktur (Objekt der Datenverarbeitung) anzeigen, insbesondere bezüglich der präoperativen Lage des Teils der Körperstruktur. Insbesondere können Verformungen des Teils der Körperstruktur (z.B. eines präoperativ bestimmten Bildobjekts im Vergleich zum intraoperativ bestimmten Bildobjekt) ebenfalls visualisiert werden. Beispielsweise kann aus der Visualisierung verformter Nervenbahnen der Bediener leichter auf die intraoperative Verformung wesentlicher Gehirnstrukturen schließen.

Insbesondere kann erfindungsgemäß das Teil der Körperstruktur (z.B. das präoperativ geplante Bildobjekt) automatisch zu der aktuellen Position im registrierten dreidimensionalen Patientenkoordinatensystem verschoben werden. Der Patient ist registriert, da erfindungsgemäß vorzugsweise mit einem Navigationssystem eine am Patienten angebrachte Markereinrichtung detektiert wird, die vorzugsweise ebenfalls von den intraoperativen und auch präoperativen Analyseverfahren erfasst wurde. Somit kann insbesondere die aktuelle Lage eines Instruments relativ zu der intraoperativen Lage des Teils der Körperstruktur angezeigt werden, wobei bei der Anzeige auf die räumlich feineren Informationen der präoperativen Daten bei der Anzeige des Teils der Körperstruktur zurückgegriffen werden kann und gleichzeitig dennoch die im Patientenkoordinatensystem angezeigte Lage des Teils der Körperstruktur der aktuell und intraoperativ bestimmten Lage entspricht.

Neben den oben genannten Verfahren können auch weitere physikalische Parameter insbesondere des Gewebes verwendet werden, beispielsweise zur Bestimmung der Elastizität, um eine Entsprechung zwischen dem intraoperativen Teil und dem präoperativen Teil zu bestimmen und insbesondere die aktuelle Form des intraoperativen Teils zu detektieren. Ein Beispiel hierfür stellt die Vibrographie dar (J Ultrasound Med 2005; 24:985-992). Die Vibrographie erlaubt die Detektion elastischer Eigenschaften des Gehirns und insbesondere eines Tumorgewebes und ermöglicht somit die Erfassung von Information, wie sie sich aus Ultraschallbildern nicht ergibt. Somit kann die Vibrographie erfindungsgemäß mit einer Ultraschalldiagnose kombiniert werden, um somit intraoperativ die Grenzen des Teils der Körperstruktur (z.B. Grenzen des Tumors) besser bestimmen zu können. Die Vibrographie erlaubt insbesondere nicht scharf abgegrenzte Teile von Körperstrukturen (also beispielsweise unscharf abgegrenzte Tumorbereiche) anzuzeigen, wenn die Unterscheidung zwischen Tumor und Gehirn nicht klar möglich ist. Kombiniert man somit erfindungsgemäß die Daten aus verschiedenen medizinischen Analyseverfahren, um aus dieser Kombination insbesondere intraoperative Daten zu erhalten, so ist eine genauere Bestimmung von Teilen der Körperstruktur und insbesondere von Substrukturen erfindungsgemäß möglich.

Vorteilhaft wird die Gewinnung der intraoperativen Daten so gestaltet, dass insbesondere oder ausschließlich die wesentlichen Strukturen, die für die Operation entscheidend sind und die insbesondere präoperativ definiert wurden, durch die intraoperativ eingesetzte medizinische Analysevorrichtung erfasst werden. Durch das erfindungsgemäße Verfahren wird es dem Bediener, insbesondere Chirurgen möglich, die Bewegung der wichtigen Strukturen (Teile der Körperstruktur) im Vergleich zu der Situation bei der präoperativen Planung zu erkennen. Vorteilhaft wird durch die Erfindung die von der Planung zur intraoperativen Situation stattfindende Lageänderung quantifiziert und diese quantifizierte Information kann vom Bediener verwendet werden, um die aktuelle Vorgehensweise im Vergleich zur geplanten zu modifizieren. Auch ist eine Verwendung der so gewonnenen Daten für eine nach dem operativen Eingriff geplante Strahlenbehandlung möglich. Vorteilhaft werden durch die vorliegende Erfindung Deformationen oder Größenänderungen von Teilen der Körperstruktur für den Bediener visualisiert. Insbesondere kann die Form eines vorab geplanten Objekts korrigiert werden. Insbesondere können Farbkodierungen eingesetzt werden, um Formveränderungen zu visualisieren. Vorteilhaft erfolgt die Anzeige und Anpassung von geplanten Objekten (präoperativen Teilen) automatisch an die aktuelle Lage bei der Visualisierung des Teils der Körperstruktur. Vorteilhaft werden Teile, die eine starre relative Beziehung zu den präoperativen Daten haben, die also insbesondere eine starre räumliche Beziehung zu einer an der Körperstruktur angebrachten Markereinrichtung haben und intraoperativ durch die medizinische Analyseeinrichtung erkannt werden, genutzt, um die Registrierung insbesondere von Instrumenten zu verfeinern.

Vorteilhaft werden Biegungen und Verformungen von Nervenbahnen, die beispielsweise durch intraoperativ eingesetztes DTI gemessen werden, genutzt, um Gehirnverformungen zu schätzen.

Vorteilhaft werden die intraoperativ eingesetzten medizinischen Analyseverfahren verwendet, um präoperativ gewonnene Daten zu ergänzen. Vorteilhaft wird also eine Kombination aus prä- und intraoperativ gewonnenen Daten angezeigt, wobei die Lage eines Teiles oder einer Substruktur der präoperativen Daten und/oder eines aus den präoperativen Daten abgeleiteten Objekts, das das Teil oder die Substruktur darstellt, erfindungsgemäß geändert wird, um mit der intraoperativen Situation übereinzustimmen. Beispielsweise kann die Kombination von Ultraschall-Daten, Dopplersonographie-Daten und Vibrographie-Daten zusätzliche Information über Tumorgrenzen liefern, wie sie sich aus präoperativ gewonnenen MRI-Bildern nicht ergaben. Damit kann sichergestellt werden, dass durch die Operation der gesamte Tumor erfasst wird. Somit kann durch den Einsatz unterschiedlicher Messtechniken (Ultraschall, MR, CT,...) und Aufnahmeparameter (US B-Mode, Vibrographie, oder MR T1,T2,DTI) mehr Information gewonnnen werden.

Im Folgenden werden bei der detaillierten Beschreibung von Ausführungsformen weitere Vorteile und Merkmale der Erfindung offenbart. Verschiedene Merkmale unterschiedlicher Ausführungsformen können miteinander kombiniert werden.

Bei der Verwendung verschiedener Analysetechniken, um Bilder einer Körperstruktur zu erzeugen, können, abhängig von der Abbildungstechnik, Grenzen von Teilen oder von Substrukturen der Körperstruktur gemessen werden oder aus den Bilddaten, die beispielsweise mittels DTI erhalten wurden, berechnet werden. Diese Eigenschaften der Substruktur werden vorzugsweise präoperativ genutzt, um automatisch (beispielsweise mittels Kantendetektion) oder manuell ein dreidimensionales Objekt, das dem präoperativen Teil oder der präoperativen Substruktur entsprechen kann, im präoperativen Datensatz festzulegen. Beispielsweise können die Grenzen eines Tumors basierend auf einem MRI-Datensatz oder die Grenzen von Nervenbahnen basierend auf einem DTI-Datensatz präoperativ festgelegt werden.

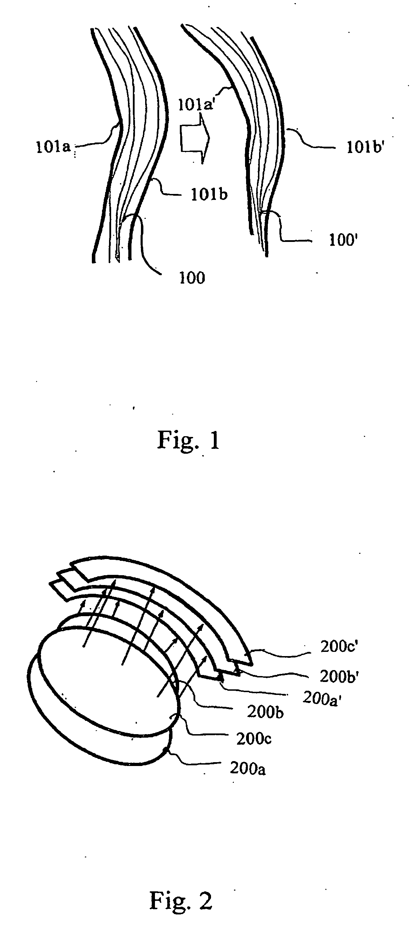

Figur 1 zeigt schematisch die Formveränderung von Nervenbahnen 100 und 100', wobei 100 die präoperative Situation darstellt und 100' die intraoperative Situation. Mit dicken schwarzen Linien 101 und 101' sind die präoperativen und intraoperativen Grenzen der Nervenbahnen hervorgehoben, die beispielsweise durch Kantendetektion aus den präoperativen Daten gewonnen werden können und die sich intraoperativ beispielsweise durch DTI-Bilder ergeben. Die Grenzen werden präoperativ vorzugsweise auch über DTI gewonnen. Eine intraoperative Kantendetektion entlang der bereits berechneten und segmentierten Faserbündel hilft insbesondere dabei, die Anpassung algorithmisch einfacher durchzuführen. Ein Anpassen der Kanten ist einfacher und schneller zu berechnen. Vorzugsweise wird die Gesamtveränderung angenommen als elastische Verformung analog zu den verformten Kanten. Die Kantendetektion ist insbesondere ein Mittel zur vereinfachten Objektanpassung. Insbesondere im Ultraschall ermöglicht sie es, einfache ObjektEigenschaften zur Anpassung zu erhalten. Die Kantendetektion wird optional auf die intraoperativen Daten angewendet. Präoperativ verläuft die Segmentierung vorzugsweise aber nicht obligatorisch mit (aufwändigeren) anderen (Standard-)Verfahren (atlasbasiert, Region Growing, Thresholding, MagicWand,....) oder manuell.

Wie man erkennt, ist der Verlauf und insbesondere die Krümmung der präoperativen Grenzen 101 a und 101a' sowie 101b und 101b' ähnlich. Das Krümmungsverhalten kann somit genutzt werden, um automatisch im intraoperativen Datensatz die Grenzen zu erkennen. Weiter kann bei der automatischen Erkennung auf die relative Lage der Grenzen 101a zu 101b zurückgegriffen werden und auf die absolute Lage der Nervenbahnen relativ zu einer Markereinrichtung.

Insgesamt kann die Ähnlichkeit der Formen des intraoperativen Teils im Vergleich zum präoperativen Teil genutzt werden, um eine automatische Objekterkennung durchzuführen. Insbesondere können für einen Bediener die Grenzen eines Teils der Körperstruktur hervorgehoben werden, beispielsweise mittels Kantendetektion, um ihm das Erkennen des entsprechenden Objekts im Bild, das auf intraoperativen Daten beruht, zu erleichtern.

Ist das intraoperative Teil bestimmt, das dem präoperativen Teil entspricht, erkannt, ist also ein anatomisches Objekt aus dem intraoperativen Datensatz extrahiert, so kann nun eine mögliche Lageverschiebung erkannt werden.

Figur 2 zeigt einen Fall, bei dem die präoperativ genutzte medizinische Analysetechnik sich von der intraoperativ genutzten Analysetechnik unterscheidet. 200a, 200b und 200c zeigen eine präoperativ bestimmte Substruktur eines Tumors, aus darstellungstechnischen Gründen vereinfacht als Scheiben des Tumors dargestellt, die zusammengenommen ein dreidimensionales Tumorobjekt bilden. Die Unterteilung eines Objekts in mehrere z.B. scheibenförmige Unterobjekte (Schichten) erleichtert auch das Erkennen einer Formänderung (siehe unten). Sie ist aber rein optional und es kann auch ein einziges dreidimensionales Objekt verwendet werden.

Die gewählte Schichtdarstellung kann auch die Bildmodalität berücksichtigen, wenn Ultraschall, MR und CT die Daten schichtweise aufnehmen.

Die Grenzen eines Teils dieser (hier im Ultraschall abgeschatteten) Substruktur werden im Ultraschall erkannt und sind schematisch durch Grenzflächen 200a', 200b' und 200c' in Figur 2 gezeigt. Die Grenzflächen 200a', 200b' und 200c' stellen Beispiele für einen intraoperativen Teil der Körperstruktur dar, der wiederum Bestandteil einer Substruktur ist. Die Substruktur stellt hier die Grenzfläche des Tumors 200 dar. Vorteilhaft wird also basierend auf den präoperativen Daten beispielsweise eines der oben erwähnten Standardverfahren oder z.B. ein Kantendetektionsalgorithmus zur Segmentierung angewendet, um so die Grenzfläche der Scheiben 200a, 200b und 200c zu bestimmen. Diese Grenzfläche ist dann die Substruktur. Zumindest ein Teil dieser Grenzfläche soll dann mit den Grenzflächen 200a' und 200b' und 200c' in Übereinstimmung gebracht werden. Ist eine Lageverschiebung des Tumors von der präoperativen Situation zur intraoperativen Situation eingetreten, so kann die vorgenannte Übereinstimmung dadurch erzielt werden, dass die Schreiben 200a, 200b und 200c wie durch Pfeile in Figur 2 angedeutet, verschoben werden, bis die Grenzen der Scheiben in die Grenzflächen 200a', 200b' und 200c' fallen. Eine damit einhergehende Verlagerung und/oder Verformung des Tumors 200 kann somit quantitativ erfasst werden. Die Größe der Verlagerung ergibt sich aus der Länge der in Figur 2 gezeigten Pfeile. Eine Verformung kann z.B. dann festgestellt werden, wenn sich für die einzelnen Scheiben eine unterschiedliche Verlagerung ergibt. Eine Verformung kann auch aus einer abweichenden Krümmung der Grenzflächen abgeleitet werden, wie weiter unten noch näher erläutert wird.

Vorteilhafterweise wird vor Anpassung der durch die Segmentierung (oder andere Standardverfahren z.B. Kantenextraktion) aus den präoperativen Daten ermittelten Grenzflächen an die intraoperativen Grenzflächen ein Ergebnis des intraoperativ genutzten medizinischen Analyseverfahrens simuliert. Beispielsweise ist bei der Ultraschalluntersuchung eines Tumors bekannt, dass es zu einer Abschattung desjenigen Teils des Tumors kommen kann, der der Ultraschallsonde abgewandt ist und nicht zugewandt ist. Somit kann man als Zwischenschritt zu dem in Figur 2 gezeigten Verfahren aus dem sich aus den Kernspindaten ergebenden Form- und Lagedaten des Tumors 200 entsprechende Ultraschallsignale simulieren. Die simulierten Ultraschallsignale ähneln dann den sichelförmigen Grenzflächen 200a' und 200b' und 200c', die in Figur 2 gezeigt sind. Diese sichelförmigen, simulierten Grenzflächen müssen dann nur noch durch Form- und Lageänderung in Übereinstimmung mit den Grenzflächen 200a', 200b' und 200c' gebracht werden.

Das vorgenannte Verfahren wurde so beschrieben, dass eine Unterteilung in zweidimensionale Scheiben erfolgt. Das genannte Verfahren kann natürlich auch ohne Unterteilung in Scheiben, also mit dreidimensionalen präoperativen Objekten, die Teile oder Substrukturen der Körperstruktur darstellen, durchgeführt werden, um festzustellen, welche Lage- oder Formänderung zur besten Übereinstimmung des präoperativen Objekts mit dem dreidimensionalen intraoperativen Objekt führt. Zur Lageänderung wird vorzugsweise eine lineare Transformation eingesetzt, die insbesondere eine Translation und/oder Rotation umfasst.

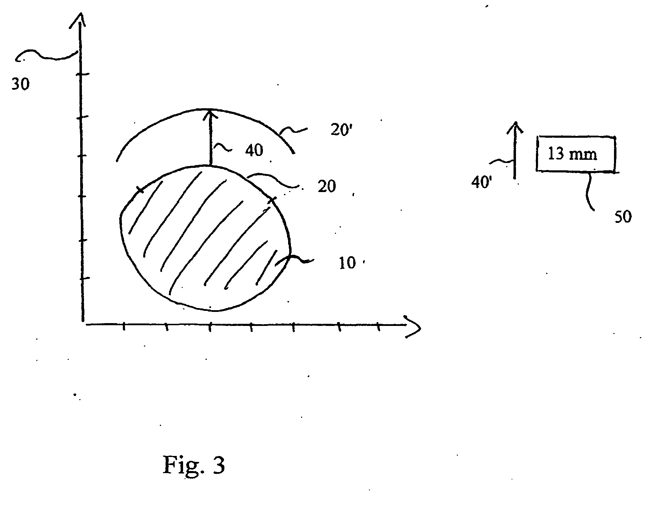

Vorzugsweise wird die Form- und/oder Lageänderung visualisiert. Ein Beispiel für eine Visualisierung einer Lageänderung zeigt Figur 3. Eine optionale Skala 30 ist eingezeigt, um dem Bediener eine Einschätzung der Lageänderung zu ermöglichen. Ein präoperatives Objekt 10, das einer präoperativen Substruktur entspricht ist angezeigt. Ein intraoperatives Objekt, das einem intraoperativen Teil der Körperstruktur entspricht, hat das Bezugszeichen 20'. Die Lage des Objekts 10 und des Objekts 20' unterscheiden sich. Mittels einer Transformation, die durch einen Pfeil 40 auf dem Bildschirm angezeigt wird, kann eine Übereinstimmung eines Abschnitts 20 der Grenze des Objekts 10 mit dem Objekt 20' erzielt werden. Der Abschnitt 20 ist der präoperative Teil der Körperstruktur, der dem intraoperativen Teil 20' entspricht. Wie in Figur 3 rechts gezeigt, kann am Bildschirm auch die Länge des Verschiebevektors 40 als separater Pfeil 40' angezeigt werden. Der Wert 50 oder der Betrag der Verschiebung kann ebenfalls getrennt in einem Bereich des Bildschirms angezeigt werden. Im gegebenen Beispiel 13 mm.

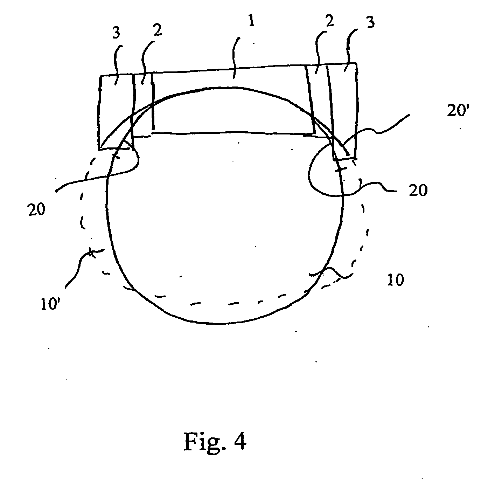

Figur 4 zeigt ein Beispiel für eine überlappende Darstellung eines präoperativen Objekts 10 und eines intraoperativen Objekts 20'. Das Objekt 10 kann vor der Darstellung in Figur 4 bereits durch eine Transformation verschoben worden sein, um eine zumindest teilweise Überlagerung bzw. Überdeckung des Objekts 10 mit dem Objekt 20' zu erzielen (die einer zumindest teilweisen Überlagerung des präoperativen Teils 20 mit dem intraoperativen Teil 20' entspricht) und um so eine Formänderung leichter für einen Bediener erkennbar zu machen. Vorzugsweise wird eine maximal mögliche Überdeckung des Randes oder Volumens angestrebt. Die Lageänderung kann in Fig. 4 dann zusätzlich durch einen Pfeil 40' oder einen Lageänderungswert 50, wie in Fig. 3 angezeigt werden.

Bei der Visualisierung der Deformation, wie sie in Figur 4 gezeigt ist, werden vorzugsweise Farbkarten eingesetzt, die verschiedene Farben je nach Umfang der Deformation zeigen. Beispielsweise kann der Bereich 1 grün sein, um so anzuzeigen, dass in diesem Bereich 1 keine Deformation detektiert wird. Der Bereich 2 kann gelb sein, um anzuzeigen, dass in diesem Bereich eine leichte Deformation detektiert wurde. Schließlich kann der Bereich 3 rot sein, um anzuzeigen, dass in diesem Bereich eine starke Deformation detektiert wurde. Zusätzlich kann automatisch ein Objekt 10' berechnet werden. Der Rand des Objekts 10' ist in Fig. 4 gestrichelt gezeichnet und umfasst außerdem den Abschnitt 20'. Das Objekt 10' kann z.B. unter der Annahme berechnet werden, dass das Objekt 10 elastisch verformbar ist und unter der Bedingung, dass der Abschnitt 20 mit dem Abschnitt 20' vollständig in Übereinstimmung gebracht wird.

Deformationen des Teils einer Körperstruktur und somit Formunterschiede zwischen präoperativen und intraoperativen Objekten können also durch Farbkodierungen am Bildschirm hervorgehoben werden. Derartige Formänderungen können z.B. durch eine Verschiebung des Gehirns auftreten oder können darin begründet sein, dass sich die medizinischen Analysetechniken unterscheiden, so dass beispielsweise intraoperativ Bereiche des Tumors erfasst werden, die präoperativ noch nicht erkannt wurden. Somit kann durch die Hervorhebung der Unterschiede, beispielsweise durch Farbcodes (oder durch verschieden frequentes Blinken) der Operateur darauf hingewiesen werden, dass es Abweichungen von seiner Planung gibt. So wird es dem Operateur erleichtert beispielsweise den gesamten Tumor zu erfassen.

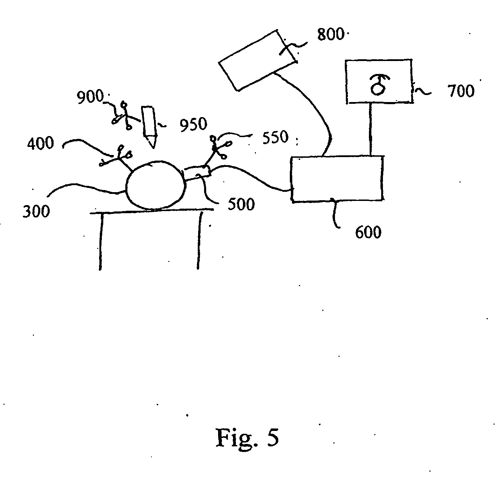

Figur 5 zeigt schematisch den Aufbau einer erfindungsgemäßen Vorrichtung. Der Kopf eines Patienten 300 ist mit einer Markereinrichtung 400 versehen, die beispielsweise am Schädel ortsfest befestigt ist. Eine Ultraschalleinrichtung 500 ist im Kontakt mit dem Kopf des Patienten, um intraoperative Daten zu gewinnen. An ihr ist ebenfalls eine Markereinrichtung 550 angebracht, um die Ultraschalleinrichtung 500 navigieren zu können. Die Ultraschalleinrichtung 500 ist mit einer Datenverarbeitungseinrichtung 600 verbunden, die auf einem Monitor 700 Hinweisinformationen beispielsweise gemäß Figur 3 oder 4 ergibt.

Eine Markerdetektionseinrichtung 800 erlaubt die Detektion der Markereinrichtung 400 und der Markereinrichtung 900, die an dem Instrument 950 angebracht ist und gibt die Detektionssignale an die Datenverarbeitungseinrichtung 600. Diese kann somit insbesondere auch die Lage des Instruments oder der Instrumentenspitze relativ zu der intraoperativen Lage des Teils (z.B. Objekt 20 in Figur 3 oder 4) der Körperstruktur berechnen. Geht man davon aus, dass beispielsweise die Anzeige gemäß Figur 4 erfolgt, so kann der Operateur erkennen, ob er mit seinem Instrument in den kritischen Bereichen 3 liegt, in denen eine Deformation stattgefunden hat und in denen er somit von seiner Planung abweichen muss. Außerdem kann er anhand des Objekts 10 erkennen, welchen Raum die Substruktur (der Tumor) in etwa einnimmt, obwohl aus den intraoperativen Daten nur ein Teil direkt ableitbar ist. Durch die Berücksichtigung der Lageänderung der Substruktur von der präoperativ geplanten Situation zu der intraoperativen Situation kann das Risiko vermindert werden, dass gesundes Gewebe beschädigt wird und die Wahrscheinlichkeit erhöht werden, dass krankes Gewebe vollständig entfernt wird.

## Patentansprüche

1. Verfahren zum Bestimmen und/oder Erkennen einer Lageänderung eines Teils (20; 100) einer Körperstruktur relativ zu einer Markereinrichtung (400) mittels einer Datenverarbeitungseinrichtung, mit folgenden Schritten:
präoperative Daten der Körperstruktur, die Informationen über die präoperative Lage des Teils (20; 100) der Körperstruktur relativ zu der Markereinrichtung (400) umfassen, werden bereitgestellt;
intraoperative Daten der Köperstruktur, die Informationen über die intraoperative Lage des Teils (20; 100') der Körperstruktur relativ zur Markereinrichtung (400) umfassen, werden bereitgestellt;
es wird basierend auf der präoperativen Lage des Teils (20; 100) und intraoperativen Lage des Teils (20'; 100') automatisch bestimmt, ob sich die Lage des präoperativen Teils (20; 100) von der Lage des intraoperativen Teils (20'; 100') unterscheidet und/oder die präoperative Lage des Teils (20; 100) und die intraoperative Lage des Teils (20'; 100') wird automatisch so angezeigt, dass eine unterschiedliche Lage erkennbar ist; und
es wird basierend auf den präoperativen Daten automatisch erkannt, ob das präoperative Teil (20) ein Unterteil einer Substruktur (10) der Körperstruktur ist, die größer als das präoperative Teil (20) ist und dieses umfasst,
**dadurch gekennzeichnet, dass**
eine Formänderung des Teils basierend auf der präoperativen Form des Teils (20) und der intraoperativen Form des Teils (20') automatisch bestimmt wird und basierend auf der bestimmten Formänderung und der präoperativen Form der Substruktur (10) eine geänderte Form der Substruktur (10') basierend auf plastischen oder elastischen Eigenschaften der Substruktur (10) und/oder auf der Annahme, dass das Volumen oder die Fläche, das oder die die Substruktur (10) einnimmt, konstant geblieben oder sich nur in einem bestimmten Umfang geändert hat, bestimmt wird.

2. Verfahren nach dem vorgehenden Anspruch, bei welchem, falls bestimmt wird, dass sich die Lage unterscheidet, automatisch entsprechende Hinweisinformation (Fig. 3) ausgegeben wird.

3. Verfahren nach einem der vorhergehenden Ansprüche, bei welchem, falls bestimmt wird, dass sich die Lage unterscheidet, die Lageänderung automatisch berechnet wird und Hinweisinformation (Fig. 3) ausgegeben wird, die über den Umfang der Lageänderung informiert.

4. Verfahren nach einem der vorhergehenden Ansprüche, bei welchem ein Bild basierend auf den präoperativen Daten angezeigt wird und zumindest einer der folgenden Schritte durchgeführt wird:
in dem Bild wird die Lage des präoperativen Teils und die Lage des intraoperativen Teils angezeigt (Fig. 3); und/oder
basierend auf der Lageänderung des Teils wird eine neue Lage des präoperativen Teils berechnet und das präoperative Teil wird alternativ zur ursprünglichen Lage oder zusätzlich dazu mit geänderter Lage im Bild angezeigt (Fig. 4); und/oder
der Umfang der Lageänderung wird im Bild bildlich hervorgehoben (Fig. 3).

5. Verfahren nach einem der vorhergehenden Ansprüche, bei welchem basierend auf den intraoperativen Daten und präoperativen Daten das dem präoperativen Teil (20; 100) entsprechende intraoperative Teil (20'; 100') automatisch bestimmt wird und/oder eine Entsprechung automatisch überprüft wird.

6. Verfahren nach dem vorhergehenden Anspruch, bei welchem die präoperative Daten der Körperstruktur Informationen über die präoperative Form des Teils der Körperstruktur umfassen, und die
intraoperative Daten der Köperstruktur Informationen über die intraoperative Form des Teils der Körperstruktur umfassen;
basierend auf einem Vergleich der präoperativen und intraoperativen Form des Teils wird automatisch bestimmt und/oder überprüft, ob das präoperative Teil dem intraoperativen Teil entspricht.

7. Verfahren nach einem der beiden vorhergehenden Ansprüche, bei welchem zum automatischen Bestimmen, ob das präoperative Teil (20; 100) dem intraoperativen Teil (20'; 100') entspricht, zumindest eines der folgenden Schritte durchgeführt wird:
ein Matching- bzw. Mustererkennungsverfahren und/oder Maximum-Entropie-Verfahren wird angewendet, bei welchem das präoperative Teil mit dem intraoperativen Teil verglichen wird; und/oder
basierend auf den präoperativen Daten der Körperstruktur wird ein Ergebnis eines intraoperativen Analyseverfahrens, das zur Gewinnung der intraoperativen Daten eingesetzt wurde, modelliert und das Ergebnis wird mit den intraoperativen Daten verglichen; und/oder
Transformationen zur iterativen Maximierung einer Überdeckung des präoperativen Teils mit dem intraoperativen Teil werden eingesetzt.

8. Verfahren nach einem der vorhergehenden Ansprüche, bei welchem eine Formänderung des Teils basierend auf der präoperativen Form des Teils (20) und der intraoperativen Form des Teils (20') automatisch bestimmt wird und bei welchem die Formänderung insbesondere mittels Farben, hervorgehoben wird (Fig. 4).

9. Verfahren nach einem der drei vorhergehenden Ansprüche, bei welchem basierend auf der Ähnlichkeit der präoperativen Lage zur intraoperativen Lage des Teils und/oder der Ähnlichkeit der Lagebeziehung zwischen mindestens zwei präoperativen Teilen (101a, 101b) zur Lagebeziehung zwischen mindestens zwei intraoperativen Teilen (101a', 101b'), automatisch bestimmt und/oder überprüft wird, ob das präoperative Teil (100) dem intraoperativen Teil (100') entspricht.

10. Verfahren nach einem der vorhergehenden Ansprüche, bei welchem automatisch darüber informiert wird (40), dass die präoperative Lage der Substruktur (10) von der intraoperativen Lage verschieden ist, falls die präoperative Lage des Teils (20) von der intraoperativen Lage des Teils (20') verschieden ist.

11. Verfahren nach dem vorhergehenden Anspruch, bei welchem, falls die Lage des präoperativen Teils von der Lage des intraoperativen Teils verschieden ist, aus der präoperativen Lage des Teils und der intraoperativen Lage des Teils eine Lageänderung automatisch berechnet wird (50);
basierend auf der berechneten Lageänderung und der präoperativen Lage der Substruktur eine intraoperative Lage der Substruktur (10) automatisch berechnet wird und die intraoperative Lage der Substruktur (10) automatisch angezeigt wird (Fig. 4).

12. Verfahren nach dem vorhergehenden Anspruch, bei welchem geometrische Eigenschaften der Substruktur basierend auf den präoperativen Daten automatisch bestimmt werden, diese geometrischen Eigenschaften als nicht oder nur in einem vorbestimmten Umfang veränderlich angenommen werden, und basierend auf dieser Annahme die Formveränderung der Substruktur bestimmt wird.

13. Verfahren nach einem der beiden vorhergehenden Ansprüche, bei welchem zur Berechnung der Formänderung der Substruktur davon ausgegangen wird, dass die Substruktur elastisch verformbar ist.

14. Programm, das, wenn es auf einem Computer läuft oder in einen Computer geladen wird, den Computer veranlasst, das Verfahren nach einem der vorhergehenden Ansprüche auszuführen.

15. Vorrichtung zum Bestimmen und/oder Erkennen einer Lageänderung eines Teils einer Körperstruktur relativ zu einer zur Körperstruktur ortsfesten Markereinrichtung
mit einer medizinischen Analyseeinrichtung (500) zum Erfassen intraoperativer Daten der Körperstruktur, die Informationen über die intraoperative Lage des Teils (20') der Körperstruktur relativ zu der Markereinrichtung (400) umfassen,
eine Datenspeichereinrichtung, die präoperative Daten der Körperstruktur speichert, die Informationen über die präoperative Lage des Teils (20) der Körperstruktur relativ zu der Markereinrichtung (400) umfassen,
einer Datenverarbeitungseinrichtung (600), auf der das Programm nach dem vorhergehenden Anspruch geladen ist oder läuft.

16. Navigationssystem
mit einer Vorrichtung nach Anspruch 15
mit einem Instrument (900), an dem eine weitere Markereinrichtung (950) angebracht ist,
mit einer Markerdetektionseinrichtung (800) zum Detektieren der ortsfesten Markereinrichtung (400) und der weiteren Markereinrichtung (950) und zum Eingeben von auf der Detektion basierenden Detektionssignalen in die Datenverarbeitungseinrichtung (600);
wobei die Datenverarbeitungseinrichtung ausgebildet ist, die Lage des Instruments (950) basierend auf den Detektionssignalen relativ zu der intraoperativen Lage des Teils (20') der Körperstruktur anzuzeigen, wobei zur Darstellung des Teils (20') oder einer Substruktur (10'), die das Teil beinhaltet, zumindest auch auf die im Vergleich zu den intraoperativen Daten größere Informationsfülle der präoperativen Daten zurückgegriffen wird, während zur Bestimmung der Lage und/oder Form des Teils (20') oder der Substruktur (10') zumindest auch auf die intraoperativen Daten zurückgegriffen wird, selbst wenn die intraoperativen Daten nur Informationen über das Teil (20') aber nicht über die gesamte Substruktur (10') enthalten.

## Claims

1. A method of determining and/or recognising, using a data processing device, a change in position of a part (20; 100) of a body structure relative to a marker device (400), the method comprising the following steps:
preoperative data of the body structure which comprise information about the preoperative position of the part (20; 100) of the body structure relative to the marker device (400), are provided;
intraoperative data of the body structure which comprise information about the intraoperative position of the part (20'; 100') of the body structure relative to the marker device (400) are provided;
based on the preoperative position of the part (20; 100) and the intraoperative position of the part (20'; 100') of the body structure, it is automatically determined whether the position of the preoperative part (20; 100) differs from the position of the intraoperative part (20'; 100') and/or the preoperative position of the part (20; 100) an the intraoperative part (20'; 100') is automatically displayed such that the difference in position is recognisable; and
based on the preoperative data, it is automatically determined whether the preoperative part (20) is a sub-part of a sub-structure (10) of the body structure which is larger than the preoperative part (20) and comprises the latter,
**characterised in that**
a change in shape of the part is determined automatically based on the preoperative shape of the part (20) and the intraoperative shape of the part (20'), and that a changed shape of the substructure (10') is determined based on the plastic or elastic properties of the substructure (10) and/or the assumption that the volume of the area which the substructure (10) takes up has stayed constant or has changed only to a specific extent.

2. The method according to the preceding claim, wherein a corresponding notification (Fig. 3) is automatically output if it is determined that there is a difference in position.

3. The method according to one of the preceding claims, wherein, if it is determined that there is a change in position, the change in position is automatically determined and a notification (Fig. 3) is output which informs about the extent of the change in position.

4. The method according to one of the preceding claims, wherein, an image is displayed based on the preoperative data and at least one of the following steps is executed:
the position of the preoperative part and the position of the intraoperative part is displayed in the image (Fig. 3); and/or
based on the change in position of the part, a new position of the preoperative part is calculated and the preoperative part is displayed in the image (Fig. 3) with the changed position alternatively or additionally to the original position ; and /or
the extent of the change in position is emphasized visually in the image (Fig. 3).

5. The method according to one of the preceding claims, wherein, the intraoperative part (20'; 100') corresponding to the preoperative part (20; 100) is determined automatically based on the intraoperative data and the preoperative data.

6. The method according to the preceding claim, wherein
the preoperative data of the body structure comprise information about the preoperative shape of the part of the body structure, and
the intraoperative data of the body structure comprise information about the intraoperative shape of the part of the body structure;
based on a comparison of the preoperative shape and the intraoperative shape of the part, it is automatically determined and/or checked whether the preoperative part corresponds to the intraoperative part.

7. The method according to one of the two preceding claims, wherein, at least one of the following steps is executed in order to determine automatically whether the preoperative part (20; 100) corresponds to the intraoperative part (20'; 100'):
a matching and/or pattern recognition method and/or a maximum entropy method is applied which comprises comparing the preoperative part to the intraoperative part; and/or
based on the preoperative data of the body structure, a result of an intraoperative analysis method which was used to receive the intraoperative data is modelled and the result is compared to the intraoperative data; and/or
transformations for iteratively maximizing an overlap of the preoperative part with and/or onto the intraoperative part are applied.

8. The method according to one of the preceding claims, wherein a change in shape of the part is determined automatically based on the preoperative shape of the part (20) and the preoperative shape of the part (20') and wherein the change in shape is emphasised in particular by means of colours (Fig. 4).

9. The method according to one of the three preceding claims, wherein based on the similarity of the preoperative position to the intraoperative position of the part and/or the similarity of the relative position between at least two preoperative parts (101a, 101b) to the relative position between at least two intraoperative parts (101a', 101b') is determined automatically and/or wherein it is checked whether the preoperative part (100) corresponds to the intraoperative part (100').

10. The method according to one of the preceding claims, wherein a notification is output (40) that the preoperative position of the substructure (10) differs from the intraoperative position if the preoperative position of the part (20) differs from the intraoperative position of the part (20').

11. The method according to the preceding claim, wherein, if the position of the preoperative part differs from the intraoperative position, a change in position is automatically calculated (50) from the preoperative position of the part and the intraoperative position of the part;
based on the calculated change in position and the preoperative position of the substructure an intraoperative position of the substructure (10) is automatically calculated and the intraoperative position of the substructure (10) is automatically displayed (Fig. 4).

12. The method according to the preceding claim, wherein geometric properties of the substructure are automatically determined based on the preoperative data, these geometric properties are not assumed to be variable or assumed to be variable only to a predetermined extent, and based on this assumption, the change in shape of the substructure is determined.

13. The method according to one of the two preceding claims, wherein it assumed, for calculating the change in shape of the substructure, that the substructure is elastically deformable.

14. A program which, when executed in a computer or loaded onto a computer, causes the computer to execute the method according to one of the preceding claims.

15. A device for determining and/or recognising a change in position of a part of a body structure relative to a marker device being fixedly located relative the body structure, having
a medical analysis device (500) for gathering intraoperative data of the body structure which comprise information about the intraoperative position of the part (20') of the body structure relative to the marker device (400),
a data storage device which stores preoperative data of the body structure which comprise information about the preoperative position of the part (20) of the body structure relative to the marker device (400),
a data processing device (600) onto which the program according to the preceding claim is loaded or which executes the program according to the preceding claim.

16. A navigation system, having
a device according to claim 15
an instrument (900) to which a further marker device (950) is attached,
a marker detection device (800) for detecting the fixedly located marker device (400) and the further marker device (950) and for entering of detection signals which are based on the detection into the data processing device (600);
wherein the data processing device is constituted to display the position of the instrument (950) based in the detection signals relative to the intraoperative position of the part (20') of the body structure, wherein for displaying the part (20') or a substructure (10') comprising the part, at least also the compared to the intraoperative data larger information content of the preoperative data is accessed, while for determining the position and/or shape of the part (20') or the substructure (10') at least also the intraoperative data are accessed, even if the intraoperative data contain information about the part (20') but not about the whole substructure (10').

## Revendications

1. Procédé pour déterminer et/ou reconnaître un changement de position d'une partie (20 ; 100) d'une structure corporelle par rapport à un marqueur (400) au moyen d'un dispositif de traitement de données comportant les étapes suivantes :
la mise à disposition de données préopératoires de la structure corporelle, comprenant des informations sur la position préopératoire de la partie (20 ; 100) de la structure corporelle par rapport au marqueur (400) ;
la mise à disposition de données peropératoires de la structure corporelle, comprenant des informations sur la position peropératoire de la partie (20' ; 100') de la structure corporelle par rapport au marqueur (400) ;
la détermination automatique, sur la base de la position préopératoire de la partie (20 ; 100) et de la position peropératoire de la partie (20', 100'), si la position de la partie préopératoire (20 ; 100) est différente de la position de la partie peropératoire (20', 100'), et/ou l'affichage automatique de la position préopératoire de la partie (20 ; 100) et la position peropératoire de la partie (20', 100') de telle manière qu'une différence de position est reconnaissable ; et
la détermination automatique, sur la base des données préopératoires, si la partie préopératoire (20) est une sous-partie d'une sous-structure (10) de la structure corporelle plus grande que la partie préopératoire (20) et la comprend,
**caractérisé en ce que**
un changement de forme de la partie est automatiquement déterminée sur la base de la forme préopératoire de la partie (20) et de la forme peropératoire de la partie (20') et, sur la base du changement de forme déterminé et de la forme préopératoire de la sous-structure (10), une forme modifiée de la sous-structure (10') est déterminée sur la base de propriétés plastiques ou élastiques de la sous-structure (10) et/ou de la supposition que le volume ou la surface occupés par la sous-structure (10) sont restés constants ou ne se sont modifiés que dans une certaine mesure.

2. Procédé selon la revendication précédente, lors duquel, s'il est déterminé que la position est différente, une information (fig. 3) correspondante est automatiquement émise.

3. Procédé selon l'une quelconque des revendications précédentes, lors duquel, s'il est déterminé que la position est différente, le changement de position est automatiquement calculé et une information (fig. 3) portant sur l'étendue du changement de position est émise.

4. Procédé selon l'une quelconque des revendications précédentes, lors duquel une image basée sur les données préopératoire est affichée et au moins l'une des étapes suivantes est exécutée:
l'affichage, dans l'image, de la position de la partie préopératoire et de la position de la partie peropératoire (fig. 3) ; et/ou
le calcul, sur la base du changement de position de la partie, d'une nouvelle position de la partie préopératoire et l'affichage, dans l'image, de la partie préopératoire soit uniquement dans sa position modifiée par rapport à la position d'origine ou bien dans les deux positions (fig. 4) ; et/ou
l'étendue du changement de position est mise en avant dans l'image (fig. 3).

5. Procédé selon l'une quelconque des revendications précédentes, lors duquel, sur la base des données peropératoires et des données préopératoires, la partie peropératoire (20' ; 100') correspondant à la partie préopératoire (20 ; 100) est automatiquement déterminée et/ou une correspondance est automatiquement vérifiée.

6. Procédé selon l'une quelconque des revendications précédentes, lors duquel les données préopératoires de la structure corporelle comprennent des informations sur la forme préopératoire de la partie de la structure corporelle, et
les données peropératoires de la structure corporelle comprennent des informations sur la forme peropératoire de la partie de la structure corporelle ;
la correspondance entre la partie préopératoire et la partie peropératoire est automatiquement déterminée et/ou vérifiée, sur la base d'une comparaison entre la forme préopératoire et la forme peropératoire de la partie.

7. Procédé selon l'une des deux revendications précédentes, lors duquel, pour déterminer automatiquement si la partie préopératoire (20 ; 100) correspond à la partie peropératoire (20' ; 100'), au moins l'une des étapes suivantes est exécutée :
utilisation d'un procédé de mise en correspondance ou de reconnaissance de formes et/ou d'un procédé d'entropie maximal, lors duquel la partie préopératoire est comparée avec la partie peropératoire ; et/ou
la modélisation, sur la base des données préopératoires de la structure corporelle, d'un résultat d'un procédé d'analyse peropératoire utilisé pour l'obtention des données peropératoires et la comparaison du résultat avec les données peropératoires ; et/ou
l'utilisation de transformations pour la maximisation itérative d'un recouvrement entre la partie préopératoire et la partie peropératoire.

8. Procédé selon l'une quelconque des revendications précédentes, lors duquel un changement de forme de la partie est automatiquement déterminé sur la base de la forme préopératoire de la partie (20) et de la forme peropératoire de la partie (20') et lors duquel le changement de forme est mis en avant, en particulier au moyen de couleurs (fig. 4).

9. Procédé selon l'une des trois revendications précédentes, lors duquel on détermine et/ou vérifie automatiquement, sur la base de la similarité entre la position préopératoire et la position peropératoire de la partie et/ou de la similarité entre la relation de position entre au moins deux parties préopératoires (101a, 101b) et la relation de position entre au moins deux parties peropératoires (101a', 101b'), si la partie préopératoire (100) correspond à la partie peropératoire (100').

10. Procédé selon l'une quelconque des revendications précédentes, lors duquel une information indiquant que la position préopératoire de la sous-structure (10) est différente de la position peropératoire est émise, si la position préopératoire de la partie (20) est différente de la position peropératoire de la partie (20').

11. Procédé selon l'une quelconque des revendications précédentes, lors duquel, si la position de la partie préopératoire est différente de la position de la partie peropératoire, un changement de position est automatiquement calculé (50) à partir de la position préopératoire de la partie et de la position peropératoire de la partie ;
une position peropératoire de la sous-structure (10) est automatiquement calculée sur la base du changement de position calculé et de la position préopératoire de la sous-structure, et la position peropératoire de la sous-structure (10) est automatiquement affichée (fig. 4).

12. Procédé selon l'une quelconque des revendications précédentes, lors duquel des propriétés géométriques de la sous-structure sont automatiquement déterminées sur la base des données préopératoires, l'on suppose que ces propriétés géométriques ne sont pas modifiables ou seulement dans une certaine mesure, et le changement de forme de la sous-structure est déterminé sur la base de cette supposition.

13. Procédé selon l'une des deux revendications précédentes, lors duquel, pour calculer le changement de forme de la sous-structure, l'on part du principe que la sous-structure est élastiquement déformable.

14. Programme qui lorsqu'il est exécuté sur un ordinateur ou chargé sur un ordinateur, provoque l'exécution par l'ordinateur du procédé selon l'une quelconque des revendications précédentes.

15. Dispositif pour déterminer et/ou reconnaître un changement de position d'une partie d'une structure corporelle par rapport à un marqueur fixe par rapport à la structure corporelle
avec un dispositif d'analyse médical (500) pour détecter les données peropératoires de la structure corporelle qui contiennent des informations sur la position peropératoire de la partie (20') de la structure corporelle par rapport au marqueur (400),
une mémoire de données qui stocke des données préopératoires de la structure corporelle qui contiennent des informations sur la position préopératoire de la partie (20) de la structure corporelle par rapport au marqueur (400),
un dispositif de traitement de données (600), dans lequel le programme selon la revendication précédente est chargé ou exécuté.

16. Système de navigation
avec un dispositif selon la revendication 15
avec un instrument (900) sur lequel est fixé un autre marqueur (950),
avec un dispositif de détection de marqueur (800) pour détecter le marqueur fixe (400) et l'autre marqueur (950) et pour entrer dans le dispositif de traitement de données (600) des signaux de détection basés sur la détection ;
où le dispositif de traitement de données est configuré pour afficher la position de l'instrument (950) par rapport à la position peropératoire de la partie (20') de la structure corporelle, sur la base des signaux de détection, où pour représenter la partie (20') ou une sous-structure (10') contenant la partie, l'on a au moins aussi recours à la quantité d'informations issue des données préopératoires, supérieure à celle issue des données peropératoires, tandis que pour déterminer la position et/ou la forme de la partie (20') ou de la sous-structure (10'), l'on a au moins aussi recours aux données peropératoires, même si les données peropératoires ne contiennent que des informations sur la partie (20') mais pas sur la totalité de la sous-structure (10').
